Europäisches Patentamt

European Patent Office

Office européen des brevets

(11) Veröffentlichungsnummer: **0 313 558**
**B1**

(12) **EUROPÄISCHE PATENTSCHRIFT**

(45) Veröffentlichungstag der Patentschrift: **02.01.91**

(51) Int. Cl.⁵: **A 61 M 29/02**

(21) Anmeldenummer: **87903683.8**

(22) Anmeldetag: **06.06.87**

(86) Internationale Anmeldenummer:
**PCT/DE87/00264**

(87) Internationale Veröffentlichungsnummer:
**WO 88/00071 14.01.88 Gazette 88/02**

(54) **DILATIONSKATHETER MIT EINEM AUFWEITBAREN BALLON.**

(30) Priorität: **26.06.86 DE 3621350**

(43) Veröffentlichungstag der Anmeldung:
**03.05.89 Patentblatt 89/18**

(45) Bekanntmachung des Hinweises auf die
Patenterteilung:
**02.01.91 Patentblatt 91/01**

(84) Benannte Vertragsstaaten:
**AT BE CH DE FR GB IT LI NL SE**

(56) Entgegenhaltungen:
**DE-A-3 442 736**
**GB-A-2 077 111**
**US-A-4 141 364**
**US-A-4 403 612**

(73) Patentinhaber: **BONZEL, Tassilo**
**Monnetstrasse 14**
**D-6400 Fulda (DE)**

(72) Erfinder: **BONZEL, Tassilo**
**Monnetstrasse 14**
**D-6400 Fulda (DE)**

(74) Vertreter: **Rackette, Karl, Dipl.-Phys. Dr.-Ing**
**Kaiser-Joseph-Strasse 179 Postfach 1310**
**D-7800 Freiburg (DE)**

## Beschreibung

Die Erfindung betrifft einen Dilatationskatheter mit einem aufweitbaren Ballon, der entlang einem sich durch einen Führungskatheter erstreckenden Führungsdraht in eine verengte Herzkranzarterie vorschiebbar und mit einem Inflationstubus verbunden ist, durch den zum Aufweiten des Ballons ein Medium in den Balloninnenraum eindrückbar ist, wobei der Ballon eine schlauchförmige Ballonaußenhülle und eine mit dieser wenigstens an den in axialer Richtung weisenden Rändern verbundene schlauchförmige Balloninnenhülle aufweist, zwischen denen ein mit dem Inflationstubus verbundener, im Querschnitt im wesentlichen ringförmiger Balloninnenraum ausgebildet ist, der ein in axialer Richtung beidseitig offenes Zentrallumen für den Führungsdraht umgibt.

Ein derartiger aus der DE 34 42 736 Al bekannter Dilatationskatheter verfügt über einen Schlauchabschnitt, der infolge des bei der Inflation des Dilatationskatheters auftretenden Kompressionsdruckes auf den Mantel des Schlauchabschnittes gegen den Führungsdraht angedrückt wird. Aus diesem Grunde wird beim Aufweiten der verengten Herzkranzarterie nicht nur die Herzkranzarterie entlang Ihrer Innenwand durch die Berührung mit der Ballonhülle, sondern auch das gegebenenfalls um den Führungsdraht vorhandene Ringvolumen verschlossen. Da eine Unterbrechung des Blutstromes im Herzmuskel gefährlich ist, wenn diese länger als etwa 30 bis 120 Sekunden dauert, muß das Aufweiten von Verengungen in Herzkranzgefäßen mit Hilfe des bekannten Dilatationskatheters sehr schnell durchgeführt werden, um eine Schädigung des Herzens zu vermeiden.

Aus der US 4 585 000 ist ein Dilatationskatheter zum Aufweiten von Herzkranzgefäßen bekannt, bei dessen Einsatz der Blutstrom nicht unterbrochen wird. Hierzu sind entlang dem Umfang des Katheters strömungsaufwärts und strömungsabwärts des aufweitbaren Bereiches Öffnungen vorgesehen, durch die der Blutstrom im Dilatationsbereich durch den Innenraum im Katheter umgeleitet wird. Im Innern des Aufweitungsbereichs ist eine Dilatationsvorrichtung mit einem ein Spindelgetriebe aufweisenden Aufweitmechanismus vorhanden, der über eine drehbare flexible Welle betätigbar ist. Neben dem Nachteil, daß die flexible Welle sich durch die Gesamtlänge des Katheters erstreckt und von außen durch Drehen betätigt werden muß, hat der bekannte Dilatationskatheter noch den Nachteil, daß die Vielzahl von scharfkantigen Bauteilen des Aufweitmechanismus die Gefahr einer relativ schnellen Trombosierung mit sich bringt.

In der US 4 198 981 ist ein Intrauterin-Instrumentarium mit einem ersten Ballon und einem im Abstand davon vorgesehenen zweiten Ballon beschrieben. Der erste Ballon verfügt über einen Schlauchabschnitt, der als verhältnismäßig steifes Rohr ausgebildet ist, um ein Einschnüren seines Innenlumens zu verhindern, durch das sich ein Rohr zum Aufblasen des zweiten Ballons erstreckt. Infolge der Steifigkeit des Schlauchabschnittes und der verhältnismäßig geringen Durchmesservergrößerung läßt sich eine entsprechend ausgebildete Anordnung nicht als Dilatationskatheter für Herzkranzarterien ausführen.

Die US 4 141 364 beschreibt einen expandierbaren endotrachialen oder urethralen Schlauch mit einer schlauchförmigen Ballonaußenhülle und einer schlauchförmigen Balloninnenhülle, die über eine Vielzahl paralleler sich in Längsrichtung erstreckender Rippen oder Bänder miteinander verbunden sind. Im nichtaufgeweiteten Zustand haben sowohl die schlauchförmige Ballonaußenhülle als auch die schlauchförmige Balloninnenhülle einen gewellten oder geriffelten Querschnitt. Die Rippen oder Bänder erstrecken sich dabei sternförmig zwischen den jeweiligen Erhebungen der im Querschnitt gewellten Ballonaußenhülle und Balloninnenhülle. Im aufgeweiteten Zustand bilden diese Rippen oder Gurte Versteifungen, die bei einem Dilatationskatheter zum Aufweiten verengter Herzkranzarterien unerwünscht sind, da die Herzkranzarterien eine Vielzahl von gewundenen Gefäßabschnitten aufweisen.

Ein anderer doppelwandiger Dilatationskatheter mit einer inneren und äußeren Ballonhülle, die an keiner Stelle unmittelbar miteinander verbunden sind, ist in der US 4 403 612 geoffenbart.

Der Erfindung liegt die Aufgabe zugrunde, einen Dilatationskatheter der eingangs genannten Art zu schaffen, der sich der Krümmung gewundener Gefäße gut anpaßt und der während der Behandlung einen ausreichend kontinuierlichen Blutfluß bei einem geringen Strömungswiderstand ohne die Gefahr einer Trombose gestattet.

Diese Aufgabe wird erfindungsgemäß dadurch gelöst, daß die Balloninnenhülle und die Ballonaußenhülle entlang mehreren sich in axialer Richtung erstreckenden unterbrochenen Linien oder Punkten in der Art einer Steppung direkt miteinander verbunden sind.

Der erfindungsgemäße Dilatationskatheter gestattet ein Aufweiten einer Herzkranzarterie ohne gleichzeitiges Unterbrechen des Blutflusses, wobei im Blutstrom innerhalb des Dilatationskatheters keine sich radial erstreckenden Strömungshindernisse vorhanden sind. Dadurch, daß sowohl die Balloninnenhülle als auch die Ballonaußenhülle bei der Abwesenheit eines Innendruckes kollabieren, ergibt sich ein äußerst geringer Durchmesser im nicht aufgeweiteten Zustand und eine hohe Verformbarkeit des aufweitbaren Ballons.

Bei der Erfindung sind die Ballonaußenhülle und die Balloninnenhülle an einer Vielzahl von Punkten in der Art einer Steppung miteinander verbunden, um eine Vielzahl von kissenförmigen Schwellkörpern zu bilden, die nach dem Einspritzen eines Gases oder einer Flüssigkeit dem mit einem Zentrallumen versehenen röhrenartigen oder schlauchartigen Dilatationskatheter in radialer Richtung eine ausreichend hohe Steifigkeit und Dilatationskraft verleihen, wobei jedoch eine Anpassung an gekrümmt verlaufende Gefäße aufgrund einer hohen axialen Flexibilität gewährleistet ist.

Zweckmäßige Ausgestaltungen und Weiterbildungen der Erfindung sind in den Unteransprüchen gekennzeichnet.

Nachfolgend wird ein Ausführungsbeispiel der Erfindung anhand eines in der Zeichnung dargestellten Ausführungsbeispiels näher beschrieben. Es zeigen:

Fig. 1 einen Dilatationskatheter gemäß der Erfindung im Längsschnitt und

Fig. 2 einen Dilatationskatheter gemäß der Erfindung im Querschnitt.

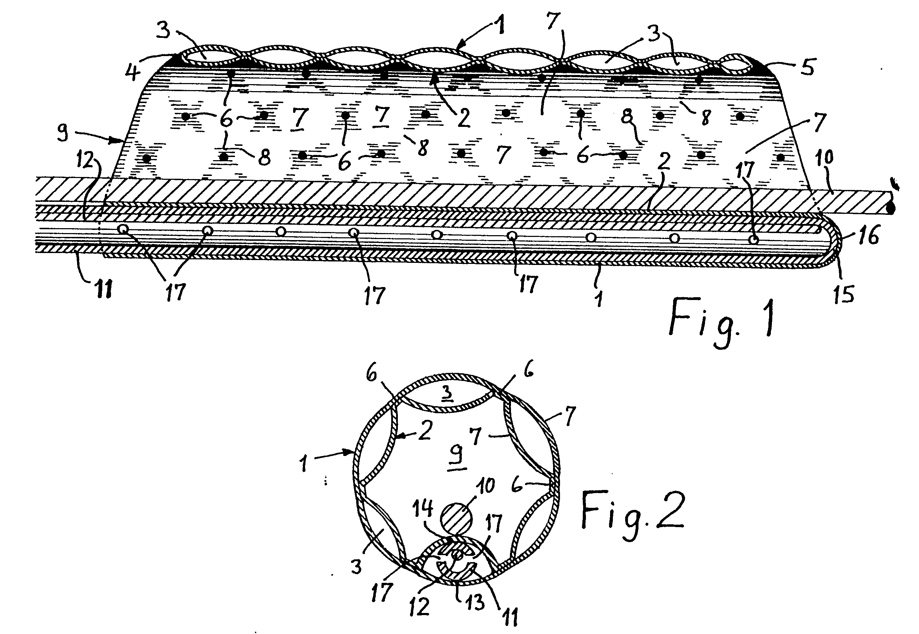

Der in Fig. 1 im Längsschnitt und in Fig. 2 im Querschnitt dargestellte doppelwandige Dilatationskatheter verfügt über eine Ballonaußenhülle 1, die je nach Bedarf aus einem elastischen oder nicht elastischen Material hergestellt ist und im wesentlichen einen äußeren Schlauch für den doppelwandigen Dilatationskatheter bildet. In etwa konzentrisch zur Ballonaußenhülle 1 ist eine Balloninnenhülle 2 vorgesehen, die je nach Bedarf aus einem elastischen oder nicht elastischen Material besteht. Die Balloninnenhülle 2 ist ebenfalls im wesentlichen schlauchförmig, wobei zwischen der Ballonaußenhülle 1 und der Balloninnenhülle 2 ein Balloninnenraum 3 gebildet ist, der beispielsweise als Ringspalt zwischen der Ballonaußenhülle 1 und der Balloninnenhülle 2 ausgebildet sein kann. Die Ballonaußenhülle 1 und die Balloninnenhülle 2 sind an ihren axialen Rändern 4, 5 miteinander verklebt oder verschweißt, so daß der Balloninnenraum 3 in axialer Richtung abgeschlossen ist.

Der auf diese Weise durch die Ballonaußenhülle 1 größeren Durchmessers und die Balloninnenhülle 2 kleineren Durchmessers gebildete rohrförmige oder schlauchförmige Ballon kann in seiner Struktur beispielsweise durch mehrere sich in axialer und/oder radialer Richtung erstreckende Verklebungen oder Verschweißungen zwischen der Ballonaußenhülle 1 und der Balloninnenhülle 2 fixiert und stabilisiert werden.

Bei dem in den Figuren 1 und 2 dargestellten Ausführungsbeispiel ist der durch die Ballonaußenhülle 1 und die Balloninnenhülle 2 gebildete rohrförmige Ballon in der Art einer Steppung durch eine Vielzahl von Punktverbindungen 6 strukturiert. An den Punktverbindungen 6 zwischen der Ballonaußenhülle 1 und der Balloninnenhülle 2 können diese miteinander verklebt oder miteinander verschweißt sein. Auf diese Weise entstehen Schwellkörper in Gestalt kissenförmiger Unterteilungen 7 des Balloninnenraums 3, wobei die einzelnen kissenförmigen Unterteilungen 7 jeweils über verbindungsfreie Bereiche 8 miteinander in Verbindung stehen, so daß eine zum Aufweiten des doppelwandigen Dilatationskatheters verwendete Flüssigkeit oder Gas zwischen den kissenförmigen Unterteilungen 7 des Balloninnenraums 3 kommunizieren kann, d.h. über die verbindungsfreien Bereiche 8 in alle kissenförmige Unterteilungen 7 des Balloninnenraums 3 vordringen kann. Die nach außen weisende Seite des ringförmigen Ballons des Dilatationskatheters und die nach innen weisende Seite des Ballons sind daher im aufgeweiteten Zustand in ihrer Oberflächenstruktur ähnlich einer Steppdecke ausgebildet. Aufgrund des Innendruckes im Balloninnenraum 3, der in allen kissenförmigen Unterteilungen 7 im stationären Zustand gleich ist, bildet der Ballon mit seiner Ballonaußenhülle 1 und seiner Balloninnenhülle 2 eine rohrartige Struktur oder die Struktur eines doppelwandigen Rohrs. Hierdurch ergibt sich eine mechanische Festigkeit und eine besondere Eigenstabilität, obwohl die Balloninnenhülle 2 ein Zentrallumen 9 offenläßt, das sich in axialer Richtung durch den Dilatationskatheter erstreckt und im aufgeweiteten Zustand des Dilatationskatheters einen Blutdurchfluß durch das aufgeweitete Gefäß gestattet.

Wie man in den Fig. 1 und 2 erkennt, erstreckt sich durch das Zentrallumen 9 ein Führungsdraht 10, der als Gleitschiene oder Leitschiene dient und entlang dem der Ballon des Dilatationskatheters durch einen üblichen schlauchartigen Führungskatheter hindurch bis zum Ort des gewünschten Eingriffs in der Herzkranzartherie vorgeschoben werden kann. Dabei ist der Balloninnenraum 3 vorzugsweise während des Vorschiebens noch nicht mit einem Füllmedium gefüllt, so daß die radiale Abmessung des Ballons des Dilatationskatheters und des Zentrallumens 9 bis zum Erreichen des Operationsortes kleiner sind als in den Figuren 1 und 2 dargestellt ist.

Wie man in den Figuren 1 und 2 erkennt, ragt ein Inflationstubus 11 exzentrisch in den Ballon des Dilatationskatheters hinein. Der Inflationstubus 11 ist versteift und überträgt innerhalb des in der Zeichnung nicht dargestellten Führungskatheters in axialer Richtung die zum Vorschieben und Zurückziehen des Ballons erforderlichen Kräfte. Zur Versteifung und zur Erhöhung der Knickfestigkeit kann im Innern des Inflationstubus 11 ein Stabilisierungsdraht 12 vorgesehen sein, der in den Fig. 1 und 2 dargestellt ist.

Der Inflationstubus 11 erstreckt sich vorzugsweise in axialer Richtung ausgehend von einem Ende 5 bis zum gegenüberliegenden Ende 4 der Balloninnenhülle 1 sowie der Ballonaußenhülle 2 und von dort über die erforderliche Länge zum Anschluß für das Füllmedium. Auf beiden Seiten neben dem Inflationstubus 11 sind im axialen Abstand mehrere miteinander fluchtende Punktverbindungen 6 vorgesehen, durch die die Lage des Inflationstubus 11 in der in Fig. 2 erkennbaren Weise im ringförmigen Zwischenraum zwischen der Ballonaußenhülle 1 und der Balloninnenhülle 2 fixiert ist. Außerdem kann der Inflationstubus 11 entlang den Berührungslinien 13, 14 mit der Ballonaußenhülle 1 und der Balloninnenhülle 2 verklebt oder verschweißt sein.

Auf der in Fig. 1 linken Seite ist der Inflationstubus 11 in einer in Fig. 1 nicht näher dargestellten Weise in Umfangsrichtung entlang seiner Außenfläche mit dem Dilatationskatheter verbunden, um den Balloninnenraum 3 nach außen hin abzudichten. Auf der in Fig. 1 rechten Seite ist der Inflationstubus 11 an seinem vorderen Ende 15 verschlossen. Entlang dem vorderen Ende 15 ist eine Verbindungswulst 16 vorgesehen, durch die Ballonaußenhülle 1 und die Balloninnehülle 2 zur Abdichtung des Balloninnenraums 3 miteinander verbunden sind.

In den Fig. 1 und 2 erkennt man, daß der Inflationstubus 11 über eine Vielzahl von Öffnungen 17 mit dem Balloninnenraum 3 in Verbindung steht, so daß ein in den Inflationstubus 11 eingepreßtes Füllmedium in die kissenförmigen Unterteilungen 7 gelangen kann, um auf diese Weise den im entleerten Zustand zusammengefalteten rohrförmigen Ballon aufzuweiten und steif werden zu lassen.

Wenn die Balloninnenhülle 2 aus elastischem Material besteht und die Ballonaußenhülle 1 aus einem nicht elastischen Material, so bewirkt dies, daß der Außendurchmesser des Ballons des Dilatationskatheters vom Druck des Füllmediums im wesentlichen unabhängig ist. Auf diese Weise wirkt die Ballonaußenhülle 1 als Haltemembran. Die elastische Balloninnenhülle 2 könnte im nicht gefüllten Zustand sehr klein sein (kleine Oberfläche) und bei der Entleerung des Ballons durch elastische Rückstellkräfte eine wesentliche Durchmesserverkleinerung bewirken. Auf diese Weise ergibt sich eine besonders leichte Passage des Dilatationskatheters beim Vorschieben oder Zurückziehen durch den Führungskatheter und/oder das jeweilige Gefäß.

Wenn statt der Balloninnenhülle 2 nur die Ballonaußenhülle 1 aus einem elastischem Material hergestellt ist, kann die Ballonweite in Abhängigkeit vom Fülldruck des Füllmediums verändert werden, wobei die Querschnittsfläche des Zentrallumens 9 bei gefülltem Ballon im wesentlichen konstant bleibt.

Wenn sowohl die Ballonaußenhülle 1 als auch die Balloninnenhülle 2 aus elastischem Material hergestellt sind, bewirkt eine Zunahme des Fülldrucks des Füllmediums überwiegend eine Dehnung der äußeren Membran, d.h. der Ballonaußenhülle 1.

Als Füllmedium können infolge des besonders kleinen Volumens des Balloninnenraums 3 schnell resorbierbare Gase wie $CO_2$ und $N_2O$ statt Flüssigkeiten benutzt werden. Während beim Platzen des Ballons des Dilatationskatheters Luftmengen von z.B. $1/10$ mm$^3$ zu Schäden führen, werden kleine Mengen $N_2O$ oder $CO_2$ von weniger als $1/10$ mm$^3$ schnell resorbiert und führen kaum zu Schäden. Dies ermöglicht es, das Lumen im Inflationstubus 11 extrem klein zu halten. Falls dies erwünscht ist, gestattet es der oben erörterte Aufbau des Dilatationskatheters, eine nur geringe Druckwirkung auf das umgebende Gewebe auszuüben. Hieraus ergibt sich, daß der Einsatz des Dilatationskatheters mit einem doppelwandigem ringförmigen Ballon besonders dann sinnvoll ist, wenn höhere Drücke nicht erforderlich sind. Dies ist der Fall bei nicht verkalkten Gefäßeinengungen und bei Stenosen, die mit Hilfe eines konventionellen Ballonkatheters bereits aufgeweitet worden sind, und zwar insbesondere dann, wenn beim Einsatz eines kein Zentrallumen aufweisenden üblichen Ballonkatheters Komplikationen aufgetreten sind. In einem solchen Fall ist es möglich, den oben beschriebenen Dilatationskatheter schnell gegen den vorher benutzten einwandigen Ballonkatheter auszuwechseln, um Komplikationen zu vermeiden oder bei eingetretenen Komplikationen eine Stabilisierung dauerhaft oder bis zum operativen Eingriff zu erreichen.

**Patentansprüche**

1. Dilatationskatheter mit einem aufweitbaren Ballon, der entlang einem sich durch einen Führungskatheter erstreckenden Führungsdraht in eine verengte Herzkranzarterie vorschiebbar und mit einem Inflationstubus verbunden ist, durch den zum Aufweiten des Ballons ein Medium in den Balloninnenraum eindrückbar ist, wobei der Ballon eine schlauchförmige Ballonaußenhülle und eine mit dieser wenigstens an den in axialer Richtung weisenden Rändern verbundene schlauchförmige Balloninnenhülle aufweist, zwischen denen ein mit dem Inflationstubus verbundener, im Querschnitt im wesentlichen ringförmiger Balloninnenraum ausgebildet ist, der ein in axialer Richtung beidseitig offenes Zentrallumen für den Führungsdraht umgibt, dadurch gekennzeichnet, daß die Balloninnenhülle (2) und die Ballonaußenhülle (1) entlang mehreren sich in axialer Richtung erstreckenden unterbrochenen Linien oder Punkten (6) in der Art einer Steppung direkt miteinander verbunden sind.

2. Dilatationskatheter nach Anspruch 1, dadurch gekennzeichnet, daß die Ballonaußenhülle (1) und die Balloninnenhülle (2) aus nichtelastischem Material bestehen.

3. Dilatationskatheter nach Anspruch 1 oder 2, dadurch gekennzeichnet, daß die Ballonaußenhülle (1) und die Balloninnenhülle (2) aus elastischem Material bestehen.

4. Dilatationskatheter nach Anspruch 1, dadurch gekennzeichnet, daß die Ballonaußenhülle (1) aus einem elastischem und die Balloninnenhülle (2) aus einem nichtelastischem Material besteht.

5. Dilatationskatheter nach Anspruch 1, dadurch gekennzeichnet, daß die Ballonaußenhülle (1) aus einem nichtelastischen und die Balloninnenhülle (2) aus einem elastischen Material besteht.

6. Dilatationskatheter nach einem der Ansprüche 1 bis 5, dadurch gekennzeichnet, daß der Inflationstubus (11) den Balloninnenraum (3) zwischen der Balloninnenhülle (2) und der Ballonaußenhülle (1) in axialer Richtung durchquert und über mehrere in den Balloninnenraum (3) mündende Öffnungen (17) verfügt.

7. Dilatationskatheter nach einem der vorstehenden Ansprüche, dadurch gekennzeichnet, daß die in axialer Richtung weisenden Ränder (4, 5) schräg gegenüber der Längsachse des Inflationstubus (11) verlaufen, wobei die axiale Länge der Ballonhülle an der freien, dem Inflationstubus (11) gegenüberliegenden Wand kleiner ist als an der mit dem Inflationstubus (11) verbundenen Wand.

**Revendications**

1. Cathéter de dilatation comportant un ballon expansible propre à être poussé, dans une artère coronaire rétrécie, le long d'un fil de guidage s'étendant dans un cathéter de guidage, et lié à un tube de gonflage par l'intermédiaire duquel un milieu peut être introduit dans l'intérieur du ballon pour élargir celui-ci le ballon présentant une enveloppe externe tubulaire et une enveloppe interne tubulaire liée à elle au moins entre ses extrémités situées en direction axiale, entre lesquelles enveloppes est ménagée une chambre interne du ballon, de section transversale essentiellement annulaire, qui est liée au tube de gonflage et qui entoure un passage central pour le fil de guidage, ouvert des deux côtés en direction axiale, caractérisé en ce que l'enveloppe interne (2) et l'enveloppe externe (1) sont directement reliées l'une à l'autre à la manière d'une couture le long de plusieurs lignes interrompues ou points s'étendant dans la direction axiale.

2. Cathéter de dilatation selon la revendication 1, caractérisé en ce que l'enveloppe externe (1) et l'enveloppe interne (2) du ballon sont constituées en un matériau non-élastique.

3. Cathéter de dilatation selon la revendication 1 ou 2, caractérisé en ce que l'enveloppe externe (1) et l'enveloppe interne (2) du ballon sont constituées en un matériau élastique.

4. Cathéter de dilatation selon la revendication 1, caractérisé en ce que l'enveloppe externe (1) du ballon est constituée en un matériau élastique et l'enveloppe interne (2) du ballon est constituée en un matériau non-élastique.

5. Cathéter de dilatation selon la revendication 1, caractérisé en ce que l'enveloppe externe (1) du ballon est constituée en un matériau non-élastique et l'enveloppe interne (2) du ballon est constituée en un matériau élastique.

6. Cathéter de dilatation selon l'une des revendications 1 à 5 caractérisé en ce que le tube (11) de gonflage traverse la chambre interne (3) du ballon dans la direction axiale, entre l'enveloppe interne et l'enveloppe externe, et en ce qu'il présente plusieurs ouvertures (17) débouchant dans la chambre interne (3) du ballon.

7. Cathéter de dilatation selon l'une des revendications précédentes caractérisé en ce que lesdites extrémités en directiuon axiale (4, 5) s'étendent obliquement par rapport à l'axe longitudinal du tube de gonflage (11), la longueur axiale de l'enveloppe du ballon sur la paroi libre opposée au tube de gonflage (11) étant plus courte que sur la paroi liée au tube de gonflage (11).

**Claims**

1. A dilation catheter with an expansible balloon which can be advanced along a guide wire extending through a guide catheter into a constricted coronary artery and is connected to an inflation tube through which a medium can be urged into the interior of the balloon to expand the balloon, wherein the balloon has a hose-like outer envelope and a hose-like inner envelope which is connected to the outer envelope at least at the edges which face in the axial direction, wherein formed between the outer and inner envelopes is an internal space which is connected to the inflation tube and which is substantially annular in cross-section and which surrounds a central lumen, which is open at both ends in the axial direction, for the guide wire, chaacterised in that the inner envelope (2) and the outer envelope (3) of the balloon are directly connected together along a plurality of points (6) or interrupted lines extending in an axial direction in the manner of quilting.

2. A dilation catheter according to claim 1 characterised in that the outer envelope (1) and the inner envelope (2) of the balloon comprise non-elastic material.

3. A dilation catheter according to claim 1 or claim 2 characterised in that the outer envelope (1) and the inner envelope (2) of the balloon comprise elastic material.

4. A dilation catheter according to claim 1 characterised in that the outer envelope (1) of the balloon comprises an elastic material and the inner envelope (2) of the balloon comprises a non-elastic material.

5. A dilation catheter according to claim 1 characterised in that the outer envelope (1) of the balloon comprises a non-elastic material and the inner envelope (2) of the balloon comprises an elastic material.

6. A dilation catheter according to one of claims 1 to 3 charcterised in that the inflation tube (1) passes in the axial direction through the internal space (3) of the balloon between the inner envelope (2) and the outer envelope (1) and has a plurality of openings (17) opening into the internal space (3) of the balloon.

7. A dilation catheter according to one of the preceding claim characterised in that the edges (4, 5) which face in the axial direction extend inclinedly with respect to the longitudinal axis of the inflation tube (11), wherein the axial length of the balloon envelope at the free wall in opposite relationship to the inflation tube (11) is less than at the wall which is connected to the inflation tube (11).

Fig. 1

Fig. 2

EP 0 313 558 B1